(19) Europäisches Patentamt
European Patent Office
Office européen des brevets

(11) Veröffentlichungsnummer: 0 311 033
A2

(12) # EUROPÄISCHE PATENTANMELDUNG

(21) Anmeldenummer: 88116444.6

(51) Int. Cl.⁴: C07D 409/04 , C07D 333/36

(22) Anmeldetag: 05.10.88

(30) Priorität: 09.10.87 DE 3734098

(43) Veröffentlichungstag der Anmeldung: 12.04.89 Patentblatt 89/15

(84) Benannte Vertragsstaaten: CH DE FR GB IT LI

(71) Anmelder: HOECHST AKTIENGESELLSCHAFT
Postfach 80 03 20
D-6230 Frankfurt am Main 80(DE)

(72) Erfinder: Litterer, Heinz, Dr.
Hardtstrasse 77
D-6208 Bad Schwalbach(DE)
Erfinder: Meidert, Helmut, Dr.
Griesheimer Stadtweg 11
D-6230 Frankfurt am Main 80(DE)
Erfinder: Hoell, Detlef, Dr.
Homberger Strasse 18
D-4130 Moers(DE)

(54) **3-Phthalimidothiophene bzw. deren Additionsprodukte mit Morpholin, Verfahren zu deren Herstellung, deren Verwendung zur Herstellung von 3-Aminothiophenen und das 3-Amino-5-chlorthiophen.**

(57) Die Erfindung bezieht sich auf 3-Phthalimidothiophene der allgemeinen Formel I

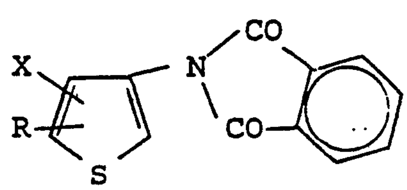

(I),

worin R Wasserstoff oder Alkyl mit 1 bis 4 C-Atomen und X Wasserstoff, Chlor oder Brom ist, bzw. deren Additionsprodukte mit Morpholin. Sie bezieht sich weiter auf ein Verfahren zur Herstellung dieser Verbindungen, bei dem man 3-Bromthiophen bzw. deren durch R und/oder X substituierten Derivaten mit Phthalimid und sekundären Aminen in gegenwart von Kupferoxyd, insbesondere Kupfer-(I)-oxyd, umsetzt und aus dem Reaktionsgemisch das Phthalimidothiophen gewinnt.

Die Erfindung bezieht sich schließlich auf die Verwendung der 3-Phthalimidothiophene der Formel I als Zwischenprodukte zur Herstellung von weitgehend bekannten 3-Aminothiophenen der allgemeinen Formel II

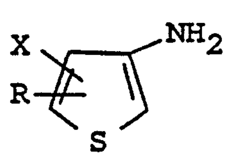

(II),

in welcher R und X die angegebene Bedeutung haben, sowie auf das 3-Amino-5-chlorthiophen.

EP 0 311 033 A2

## 3-Phthalimidothiophene bzw. deren Additionsprodukte mit Morpholin, Verfahren zu deren Herstellung, deren Verwendung zur Herstellung von 3-Aminothiophenen und das 3-Amino-5-chlorthiphen

Die Erfindung betrifft 3-Phthalimidothiophene bzw. deren Additionsprodukte mit Morpholin, ein Verfahren zu deren Herstellung, deren Verwendung als Zwischenprodukte zur Herstellung von weitgehend bekannten 3-Aminothiophenen und das 3-Amino-5-chlorthiophen. Diese 3-Phthalimidothiophene und die daraus erhältlichen 3-Aminothiophene sind wertvolle Zwischenprodukte für die Herstellung von Wirkstoffen für Arznei- und Pflanzenschutzmittel, von Farbstoffen und Polymeren.

3-Aminothiophene sind wegen ihrer Instabilität und Neigung zur Polymerisation nur schwierig zu isolieren (S. Rault u.a., J.R. Neth. Chem. Soc. 101, 205, 1982). Daher wurden sie bisher meist in Form stabiler Derivate synthetisiert und charakterisiert. So wurde das 3-Aminothiophen als Reduktionsprodukt des 3-Niitrothiophens 1933 von W. Steinkopf und T. Höpner (Ann. Chem. 501, 174, 1933) in Form des Acetyl- und Benzoylderivats erhalten. Auch E. Campaigne und P.A. Monroe (J. Am. Chem. Soc. 76, 2447, 1954) gelang durch Hofmann-Umlagerung von 3-Thenoylamid nur die Herstellung der Acyl- und Sulfonylderivate des 3-Aminothiophens. Erst E.W. Brunett u.a. konnten 1973 mittels präparativer Gaschromatographie die freie Base 3-Aminothiophen gewinnen und charakterisieren (J. Heterocycl. Chem. 10, 1067, 1973). Es liegt auf der Hand, daß nach dieser Methode nur sehr geringe Mengen des freien Aminothiophens hergestellt werden können.

Eine andere präparative Methode zur Herstellung von 3-Aminothiophenen stellt die Umsetzung von Halothiophenen mit $KNH_2$ in flüssigem $NH_3$ unter Schutzgasatmosphäre dar (J.F. Bunnett, Heterocycles 5, 377, 1976). Der Umgang mit flüssigem Ammoniak ist jedoch wegen seines niedrigen Siedepunkts und seiner Giftigkeit umständlich, gefährlich und technisch aufwendig. Darüberhinaus kann das gebildete Aminothiophen aufgrund seiner hohen Thermolabilität nur mit großen Verlusten isoliert werden.

Es bestand daher ein Bedarf an einem einfachen Verfahren zur Herstellung auch größerer Mengen an 3-Aminothiophenen. Angesichts der bekannten Empfindlichkeit der Aminothiophene ist es außerordentlich überraschend, daß es erfindungsgemäß gelingt, 3-Aminothiophene aus den gut zugänglichen 3-Phthalimidothiophenen in hohen Ausbeuten herzustellen.

Gegenstand der Erfindung sind daher neue 3-Phthalimidothiophene der allgemeinen Formel I (s. Patentanspruch 1) ein Verfahren zu deren Herstellung sowie deren Verwendung zur Herstellung von 3-Aminothiophenen. Gegenstand der Erfindung ist weiterhin das 3-Amino-5-chlorthiophen.

In den Verbindungen der Formel I bedeutet R Alkyl mit 1 bis 4 C-Atomen oder vorzugsweise Wasserstoff und X Wasserstoff, Chlor oder Brom. Die Alkylreste können geradkettig oder verzweigt sein und sind z.B. Methyl, Äthyl, n- oder Isopropyl, n-, sek.-, iso- oder tert.-Butyl.

Die Verbindungen der Formel I lassen sich vorteilhaft durch Umsetzung von 3-Bromthiophen bzw. deren durch R und/oder X substituierten Derivaten mit Phthalimid und sekundären Aminen in Gegenwart von Kupferoxyd wie Kupfer- (II)-oxyd und insbesondere Kupfer-(I)-oxyd, zweckmäßig in mindestens der stöchiometrischen Menge, herstellen. Die sekundären Amine und das Phthalimid werden in mindestens der stöchiometrischen Menge verwendet, zweckmäßig aber in einem Überschuß, der beim Phthalimid z. B. 100% und beim Amin zweckmäßig 200 bis 500 % beträgt.

Die Umsetzung wird im allgemeinen unter Atmosphärendruck durchgeführt, z.B. bei Temperaturen von 100 bis 150, vorteilhaft 120 bis 140 und insbesondere 125 bis 135 °C. Die Reaktionskomponenten können als solche eingesetzt werden, jedoch ist es vielfach vorteilhaft, Lösungs- bzw. Verdünnungsmittel, die unter den Reaktionsbedingungen gegenüber den Reaktionsteilnehmern inert sind, zu verwenden, und zwar zweckmäßig solche, die über 100 °C sieden. Derartige geeignete Lösungs-und Verdünnungsmittel sind z.B. aromatische Kohlenwasserstoffe wie Toluol, die verschiedenen Xylole und Chlorbenzol, vorzugsweise jedoch Alkohole, welcher Begriff partiell verätherte mehrwertige Alkohole einschließt. Geeignete Verbindunden sind beispielsweise die verschiedenen Butanole, Hexanole und Octanole, Äthylenglykol und Glyzerin, vorzugsweise jedoch Äthylenglykolmonomethyl- bzw. - monoäthyläther.

Als sekundäre Amine eignen sich z. B. Dipropyl- und Dibutylamin bzw. deren Isomere, Dimethylanilin, vorzugsweise jedoch cycloaliphatische Verbindunden wie Piperidin, Pyrrolidin und insbesondere das Morpholin.

Bei der Umsetzung der 3-Bromthiophene mit Phthalimid ensteht bei Verwendung von Morpholin als sekundärem Amin zunächst eine Additionsverbindung ausd dem gebilderten 3-Phthalimidothiophen und dem Morpholin. Diese ist eine schwelösliche kristalline Substanz, die aus Lösungsmitteln, wie nierderen Alkoholen, wie Methanol, Äthanol odere den Propanolen, oder Dimethylformamid, gegebenenfalls unter Zusatz von Wasser, umkristallisiert werden können. Aus ihr läßt sich in üblicher Weise durch Neutralisation mit anorganischen oder organischen Säuren, wie Chlor-oder Bromwasserstoffsäure, Schwefelsäure oder

Eisessig, bei erhöhter Temperatur das freie 3-Phthalimidothiophen der Formel I gewinnen.

Gegenstand der Erfindung ist auch die Verwendung der Verbindungen der Formel I zur Herstellung von 3-Aminothiophenen der Formel II, in der R und X die gleiche Bedeutung wie in den Verbindungen der Formel I haben. Zur Herstellung dieser 3-Aminothiophene setzt man 3-Phthalimidothiophene der Formel I mit einem Polyalkylenpolyamin mit 2 bis 6 Aminogruppen und mindestens 4 C-Atomen um, wobei die Alkyleneinheit mindestens 2 und z. B. bis zu 6 C-Atomen hat und die Stickstoffatome voneinander jeweils durch wenigstens 2 C-Atome getrennt sind.

Geeignete Polyalkylenpolyamine sind solche, die einen höheren Siedepunkt haben als das Aminothiophen. Vorteilhaft liegt der Siedepunkt mindestens 50, vorzugsweise 80 bis 150 °C höher als der des Aminothiophens. Geeignete Polyalkylenpolyamine sind z.B. Hexamethylendiamin, Dipropylentriamin, Triäthylentetramin, Tetraäthylenpentamin, Pentamethylenhexamin und Bishexamethylentriamin. Diese haben such eine ausreichende Reaktivität. Bevorzugt wird Tetraäthylenpentamin eingesetzt.

Die Reaktion kann in Gegenwart oder Abwesenheit eines hochsiedenden und unter den Reaktionsbedingungen inerten Lösungsmittels durchgeführt werden. Bevorzugt werden die Phthalimidothiophene in einem Überschuß des Polyalkylenpolyamins erhitzt, das dabei als Lösungsmittel und als Reaktionspartner dient. Das Molverhältnis des Polyalkylenpolyamins zum eingesetzten Phthalimidothiophen beträgt im allgemeinen 1:1 bis 8:1, vorzugsweise 3:1 bis 6:1.

Die Reaktionstemperatur bei der Umsetzung des 3-Phthalimidothiophens gemäß Formel I mit dem Polyalkylenpolyamin liegt zweckmäßig bei 60 bis 160 °C, vorzugsweise bei 80 bis 140 °C. Wegen der Thermolabilität der 3-Aminothiophene ist es zweckmäßig, diese bei möglichst niedrigen Temperaturen und daher bei vermindertem Druck abzudestillieren und dabei dafür zu sorgen, daß diese Verbindungen nur möglichst kurze Zeit höheren Temperaturen ausgesetzt werden.

## Beispiele

### 1) 3-Phthalimidothiophen-Morpholin-Addukt

98 g (0,6 mol) 3-Bromthiophen, 132 g (0,9 mol) Phthalimid, 200 g (2,3 mol) Morpholin, 80 ml Äthylenglykolmonomethyläther und 45 g (0,31 mol) Kupfer-(I)-oxid wurden bei Raumtemperatur in einem 1 Ltr.-Vierhalskolben unter Rühren vereinigt und unter $N_2$-Atmosphäre 90 Minuten lang bei 130 °C gerührt. Nach Erkalten auf ca. 80 °C wurde eine Mischung aus 175 ml Methanol und 10 ml Wasser in das Reaktionsgemisch eingerührt und die Kristallsuspension einige Stunden steengelassen. Anschließend wurde über eine Glasfritte abgesaugt und das kristalline Produkt auf der Fritte nach Entfernen des Unterdrucks dreimal mit je 300 ml Methanol unter Aufrühen intensiv gewaschen und so unumgesetztes Phthalimid entfernt. Man erhielt 136 g rohes 3-Phthalimidothiophen-Morpholin-Addukt (71,7 % d.Th.) als graukristalline Substanz von Schmelzpunkt 212-2142 °C, das, aus Butanol umkristallisiert, bei 219 °C schmilzt.

### 2) 3-Phthalimidothiophen

32 g (0,1 mol) des rohen Morpholin-Addukts vom Fp. 212 -214 °C wurden in einem 500 ml-Vierhalskolben mit 250 ml 1 n Salzsäure ca. 10 Min. am Rückfluß erhitzt. Die breiige Suspension wurde nach Erkhalten abgesaugt und mit Wasser neutral gewaschen. Man erhielt 20 g (87,3 % d.Th.) rohes 3-Phthalimidothiophen, das nach Kristallisation aus Eisessig bei 182 °C schmilzt.

### 3) 3-Aminothiophen

26 g (0,13) 3-Phthalimidothiophen und 100 g (0,52 mol) Tetren (Tetraäthylenpentamin, techn.) wurden in einem 250 ml-Rührkolben vereinigt. Dann destillierte man aus diesem Kolben über einen absteigenden Kühler unter Rühren und $N_2$-Überlagerung das sich beim Erhitzen nach und nach bildende 3-Aminothiopen bei gutem Vakuum kontinuierlich ab. Beginnend bei einer Sumpftemperatur von ca. 90 °C destillierten bei 50 - 70 °C/0,5 mbar unter langsamer Erhöhung der Sumpftemperatur auf 135 °C insgesamt 10 g 3-Aminothiophen über, entsprechend einer Ausbeute von 80% d.Th. Das fast farblose, ölige Destillat wurde mit $N_2$ überlagert und ist in der Kälte bei Temperaturen unter 0 °C gut haltbar.

4) 3-Phthalimido-5-chlorthiophen-Morpholin-Addukt

118, 5 g (0,6 mol) 3-Brom-5-chlorthiophen (nach anderer Nomenklatur 2-Chlor-4-bromthiophen), 132 g (0,9 mol) Phthalimid, 200 g (2,3 mol) Morpholin, 80 ml Äthylenglykolmonomethyläther und 45 g (0,31 mol) Kupfer-(I)-oxid wurden bei Raumpteratur vereinigt und unter $N_2$-Atmohsphäre 90 Min. lang bei 130 - 132° C gerührt. Nach Erkhalten auf ca. 80° C wurde eine Mischung aus 125 ml Methanol und 75 ml Wasser in das Reaktionsgemisch eingerührt und das abgeschiedene Kristallisat wie im Beispiel 1 beschrieben isoliert. Man erhielt 122 g rohes 3-Phthalimido-5-chlorthiophen-Morpholin-Addukt (58,1% d. Th.) als grünlich kristalline Substanz, die bei 177° C schmilzt.

5) 3-Phthalimido-5-chlorthiophen

Die Herstellung erfolgte analog Beispiel 2, aber mit ca. 1n äthanolischer HCl aus 225 ml Äthanol + 25 ml konz. HCl. Ausgehend von 35 g (0,1 mol) Morpholin-Addukt vom Schmelzpunkt 177° C erhielt man 24 g rohes 3-Phthalimido-5-chlorthiophen (91,2 % d.Th.), das bei 160 -163° C schmilzt, bzw. nach Kristallisation aus Aceton/Isopropanol (1:1) bei 168 - 170° C.

6) 3-Amino-5-chlorthiophen

96 g (0,36 mol) 3-Phthalimido-5-chlorthiophen (nach anderer Nomenklatur 2-Chlor-4-phthalimidothiophen und 144 g (0,75 mol) Tetren wurden in einem 500 ml-Rührkolben vereinigt. Reaktion und Aufarbeitung erfolgten analog Beispiel 3. Bei einer Sumpftemperatur von 90° C bis auf zuletzt 135° C ansteigend erhielt man eine bei 70 - 90° C/0,5 - 0,6 mbar übergehende Fraktion von 40 g 3-Amino-5-chlorthiophen, entsprechend einer Ausbeute von 82 % d.Th. Das Produkt ist unter $N_2$-Schutzgas in der Kälte bei Temperaturen unter 0° C gut haltbar.

## Ansprüche

1. 3-Phthalimidothiophene der allgemeinen Formel I

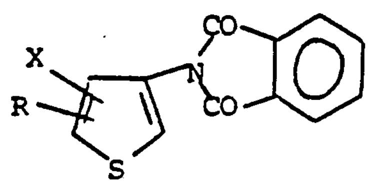

(I)

worin R Wasserstoff ode Alkyl mit 1 bis 4 C-Atomen und X Wasserstoff, Chlor oder Brom ist, bzw. deren Additionsprodukte mit Morpholin.

2. Verbindungen nach Anspruch 1, dadurch gekennzeichnet, daß X von Wasserstoff verschieden ist und in 5-Stellung steht.

3. Verfahren zur Herstellung von 3-Phthalimidothiophenen der allgemeinen Formel I gemäß Patentanspruch 1 oder 2, bzw. deren Additionsprodukte mit Morpholin, dadurch gekennzeichnet, daß man 3-Bromthiophen bzw. deren durch R und/oder X substituierte Derivate mit Phthalimid und sekundären Aminen in Gegenwart von Kupferoxyd, insbesondere Kupfer-(I)-oxyd, zweckmäßig in mindestens der stöchiometrischen Menge, umsetzt und aus dem Reaktionsgemisch das Phthalimidothiophen gewinnt.

4. Verfahren nach Anspruch 3, dadurch gekennzeichnet, daß als sekundäres Amin ein cycloaliphatisches Amin, insbesondere Morpholin verwendet wird, wobei das Umsetzungsprodukt mit Morpholin entweder als Additionsprodukt oder, nach dessen Nuetralisation, als das freie Phthalimidothiopen gewonnen wird.

5. Verfahren nach Anspruch 3 oder 4, dadurch gekennzeichnet, daß man die Umsetzung bei Temperaturen von 100 bis 150° C vorteilhaft bei 120 bis 140° C und insbesondere bei 125 bis 135° C, durchführt.

6. Verfahren nach einem oder mehreren der Ansprüche 3 bis 5, dadurch gekennzeichnet, daß die Reaktion in Gegenwart eines Lösungs- oder Verdünnungsmittels durchgeführt wird, das unter den Reaktionsbedingungen gegenüber den Reaktionsteilnehmern inert ist und vorzugsweise über 100° C siedet.

7. Verwendung von 3-Phthalimidothiophenen der allgemeinen Formel I gemäß Patentanspruch 1 oder 2 als Zwischenprodukte zur Herstellung von weitgehend bekannten 3-Aminothiophenen der allgemeinen Formel II

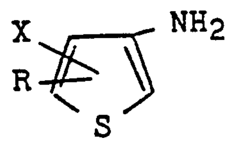

II

in welcher R und X die im Anspruch 1 bzw. 2 angegebene Bedeutung haben.

8. Ausführungsform nach Anspruch 7, dadurch gekennzeichnet, daß man ein 3-Phthalimidothiophen der Formel I gemäß Patentanspruch 1 oder 2 mit einem Polyalkylenpolyamin mit 2 bis 6 Aminogruppen und mindestens 4 C-Atomen umsetzt, wobei die Alkyleneinheit mindestens 2 und zweckmäßig nicht mehr als 6 C-Atome hat und die Sticksoffatome voneinander jeweils durch wenigstens 2 C-Atome getrennt sind, wobei das Polyalkylenpolyamin wenigstens denselben Siedepunkt hat wie das herzustellende 3-Aminothiophen und vorzugsweise Tetraäthylenpentamin ist.

9. Ausführungsform nach Anspruch 8, dadurch gekennzeichnet, daß die Umsetzung bei 60 bis 160 °C, vorzugsweise bei 80 bis 140 °C durchgeführt wird, wobei das gebildete 3-Aminothiophen vorzugsweise bei vermindertem Druck aus dem Reaktionssystem abdestilliert wird.

10. Ausführungsform nach Anspruch 8 oder 9, dadurch gekennzeichnet, daß die Umsetzung in Gegenwart eines unter den Reaktionsbedingungen gegenüber den Reaktionsteilnehmern inereten Lösungsmittels durchgeführt wird, das mindestens 50, vorzugsweise 80 bis 150 °C höher siedet als das Aminothiophen.

11. Ausführungsform nach einem oder mehreren der Ansprüche 8 bis 10, dadurch gekennzeichnet, daß das Polyalkylenpolyamin in einer solchen Menge eingesetzt wird, daß das Molverhältnis zum Phthalimidothiophen 1:1 bis 8:1, vorzugsweise 3:1 bis 6:1 beträgt.

12. 3-Amino-5-chlorthiophen.